# EUROPÄISCHE PATENTSCHRIFT

(11) **EP 0 617 645 B1**
(45) Veröffentlichungstag und Bekanntmachung des Hinweises auf die Patenterteilung: **03.04.1996**
(21) Anmeldenummer: 93918863.7
(22) Anmeldetag: 03.09.1993
(51) Int. Cl.: B09B 3/00, G01N 33/24

(54) **VERFAHREN ZUR IMMOBILISERUNG VON ORGANISCHEN UND ANORGANISCHEN SCHADSTOFFEN IN EINEM KONTAMINIERTEN BODENMATERIAL EINER SANIERUNGSFLÄCHE**
PROCESS FOR IMMOBILISING ORGANIC AND INORGANIC POLLUTANTS IN CONTAMINATED SOIL ON RECONSTRUCTION SITES
PROCEDE D'IMMOBILISATION DE POLLUANTS ORGANIQUES ET INORGANIQUES DANS LE SOL CONTAMINE D'UNE SURFACE DE RENOVATION URBAINE

(30) Priorität: 04.09.1992 CH 2782/92
(43) Veröffentlichungstag der Anmeldung: 05.10.1994
(73) Patentinhaber: KRUSE, Kai, D-53579 Erpel/rh (DE)
(72) Erfinder: KRUSE, Kai, D-53579 Erpel/rh (DE)
(86) Internationale Anmeldenummer: CH9300217
(87) Internationale Veröffentlichungsnummer: WO9405438

(56) Entgegenhaltungen:
- EP-A- 0 192 954
- US-A- 4 473 477
- JOURNAL OF THE AIR AND WASTE MANAGEMENT ASSOCIATION Bd. 40, Nr. 5 , Mai 1990 , PITTSBURGH US Seiten 704 - 732 C. SIMS 'Soil remediation techniques at uncontrolled hazardous waste sites'
- JOURNAL OF THE AIR AND WASTE MANAGEMENT ASSOCIATION Bd. 40, Nr. 11 , November 1990 , PITTSBURGH US Seiten 1569 - 1576 K. STINSON 'EPA SITE demonstration of the International Waste Technologies / Geo-Con in situ stabilization / solidification process'
- JOURNAL OF THE WATER POLLUTION CONTROL FEDERATION Bd. 58, Nr. 1 , Januar 1986 , WASHINGTON US Seiten 68 - 76 A. WOLFE 'Adsorption of organic pollutants on montmorillonite treated with amines'

## Beschreibung

Die vorliegende Erfindung betrifft ein Verfahren zur Immobilisierung von organischen und anorganischen Schadstoffen in einem kontaminierten Bodenmaterial einer Sanierungsfläche gemäss den Oberbegriffen der Patentansprüche 1 und 5.

Es ist bekannt, dass quellfähige Tonmaterialien zur Adsorption von Schwermetallionen in Abwasserbehandlungssystemen eingesetzt werden, oder dass Organo-Bentonite, welche hohe Adsorptionseigenschaften besitzen, zur Veredelung von Deponiebarrieren verwendet werden.

In der PS-US 4'473'477 wird ein Verfahren zur kontrollierten Lagerung von organische Schadstoffe enthaltenden Materialien beschrieben, welche zur Adsorption in einem organophilen Bentonit (Organoclay) eingebunden werden. Dazu wird ein organphiler Bentonit benutzt, um flüssiges oder festes schadstoffbelastetes organisches Material zu binden. Nachteilig ist, dass der ganze Müllkörper mit organophilen Bentonit versetzt werden muss, oder Sickerwasser wird durch einen Filterkörper aus organophilem Bentonit gepumpt, oder als Deponieabdichtung wird eine Lage von reinem organophilen Bentonit vorgeschlagen. Der hohe Aufwand an organophilen Bentonit ist unwirtschaftlich.

Im weiteren ist bekannt, dass Barrierenmaterial, welches eine zu grosse Durchlässigkeit aufweist und eine zu geringe Kationenaustauschkapazität besitzt oder eine zu geringe Schwermetalladsorption aufweist, durch den Zuschlag eines quellfähigen Tones, z.B. eines mit S-Trimercaptotriazin in Form des Natriumsalzes behandelten Bentonits, verbessert werden kann. Dies kann durch eine Zwangsvermischung oder einem Einfräsen oder durch Injizierung erfolgen. Dadurch kann die vierfache Adsorption gegenüber einem herkömmlichen Ca-Bentonit erreicht werden. Zudem wird die quellende Wirkung der Na-Ionen wirksam, die eine wesentliche Verringerung des Durchlässigkeitsbeiwertes bewirkt, welcher auch nach dem Eintausch der Schwermetallionen gegen die Na-Ionen konstant bleibt, da die übrigen quellfähigen Tonmaterialien diese überschüssigen Na-Ionen eintauschen.

Im weiteren sind zur Immobilisierung von Schadstoffen in Böden Verfestigungsverfahren bekannt, bei welchen der kontaminierte Boden beispielsweise mit Zement verfestigt wird. Der dadurch verfestigte Boden ist kein eigentlicher Boden mehr, sondern ein betonähnlicher Körper, welcher mit den üblichen Grundbaugeräten nicht mehr bearbeitet werden kann, was sich nachteilig auswirkt.

Nachteilig bei der Immobilisierung mit Chemikalien ist vielfach deren Toxizität, wobei bei deren Handhabung neue Kontaminationen hervorgerufen werden. Bei herkömmlichen Waschverfahren werden die Schadstoffe in verdünnter Form gewonnen und fallen z.T. durch die langen Behandlungszeiten in grossen Mengen an. Zudem ist eine herkömmliche Waschung nur bei Grobfraktionen durchführbar und nicht bei Feinfraktionen. Auch wird bei dieser Art der Waschung keine Immobilisierung der verbleibenden Schadstoffe erreicht.

Aufgabe der vorliegenden Erfindung ist es, ein Verfahren anzugeben, bei welchem ein mit Schadstoffen kontaminiertes Bodenmaterial durch eine gezielte Tonzugabe eine Immobilisierung der vorhandenen Schadstoffe bewirkt und/oder durch eine Waschung der Schadstoffgehalt verringert wird.

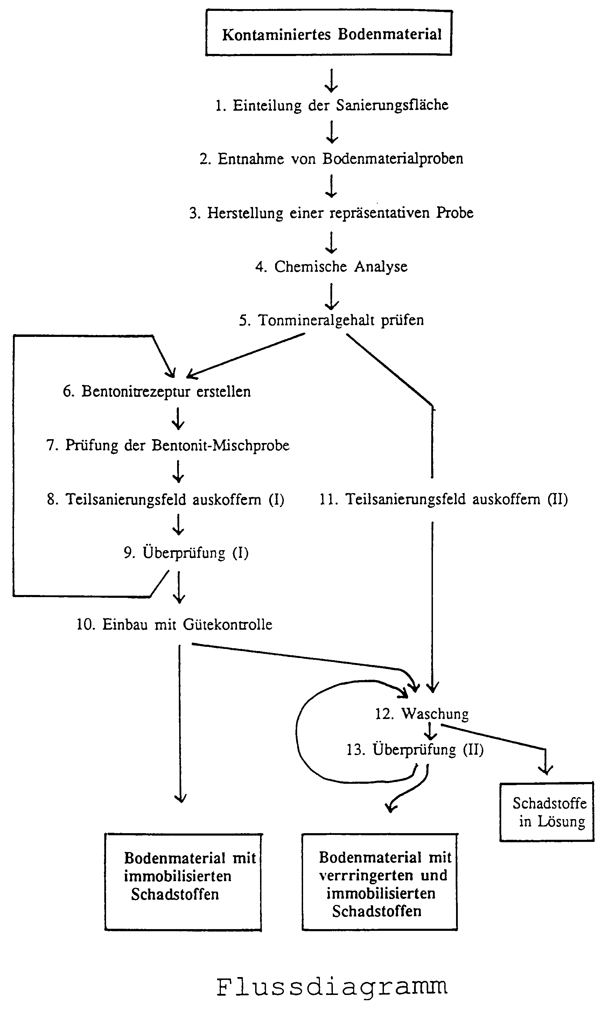

Erfindungsgemäss wird diese Aufgabe mittels Verfahren gemäss dem Wortlaut der Ansprüche 1 und 5 gelöst. Ausführungsformen der erfindungsgemässen Verfahren sind in den Ansprüchen 2 bis 4 und 6 bis 9 umschrieben. Die erfindungsgemässen Verfahren werden unter Bezug auf das Flussdiagramm (Figur) näher erläutert, wobei in der Figur die allgemeinen Schritte dargestellt sind. Dabei werden für die Beschreibung der verschiedenen Verfahrensschritte diejenigen Referenzzahlen des Flussdiagrammes verwendet, die für die Gliederung der einzelnen Schritte angeführt sind. Als Beispiel wird eine Ausführungsform dazu beschrieben. Die einzige Figur zeigt:
Ein Flussdiagramm für ein Verfahren zur Immobilisierung von Schadstoffen in kontaminiertem Bodenmaterial
Der Begriff Bodenmaterial umfasst einerseits Material gewachsener Böden im bodenkundlichen Sinne und andererseits Auffüllungsmaterialien wie beispielsweise Betonresten, Schotter und Bergematerialien, sowie Anschüttungen beliebiger Art.

Die Figur zeigt das Flussdiagramm für ein Verfahren, wonach ein mit Schadstoffen kontaminiertem Bodenmaterial gewaschen wird oder mit einem Tonmaterial veredelt und bei Bedarf ebenfalls gewaschen wird. Dieses Verfahren wird durch die Schritte 1 - 13 nachstehend beschrieben.

### 1. Einteilung der Sanierungsfläche.

Die gesamte Sanierungsfläsche wird in Abhängigkeit der Schadstoffzusammensetzung, welche vorgängig ermittelt wurde, in Teilsanierungsfelder mit einer Grösse von beispielsweise 20 x 20 m unterteilt, welche mit 1 bis i bezeichnet werden. Je homogener die Schadstoffzusammensetzung, desto grösser können die Teilsanierungsfelder gewählt werden. Bei sehr homogener Schadstoffzusammensetzung kann sich im Grenzfall eine Aufteilung sogar erübrigen, wobei dann i gleich 1 wird.

### 2. Entnahme von Bodenmaterialproben.

Aus den einzelnen Teilsanierungsfeldern werden Bodenmaterialproben gezogen, und zwar beispielsweise 8 Proben P' (I) aus einer Tiefe von 0,5 m und 8 Proben P'' (I) aus einer Tiefe von 1,0 m.

### 3. Herstellung einer repäsentativen Probe.

Aus den je 8 Proben P' (I) und P'' (I) nach Schritt 2 wird durch Mischen eine repräsentative, dem i-ten Teilsanierungsfeld zugeordnete Probe P(i) hergestellt.

### 4. Chemische Analyse.

Von jeder Probe P(i) werden nun die Art der organischen und anorganischen Schadstoffe und deren Konzentrationen ermittelt. Dies erfolgt im allgemeinen aus der Analyse der Trockensubstanz und aus den Eluatversuchen, wie beispielsweise der DEV-S4 in Deutschland. Zusätzlich werden von jeder Probe P (i) Art und Menge der vorliegenden Tonmaterialien bestimmt, woaus sich ein Tonmineralgehalt bestimmen lässt.

### 5. Tonmineralgehalt prüfen.

Liegt der in Schritt 4 bestimmte Tonmineralgehalt unterhalb 60 Gew.-% wird das Verfahren mit den Schritten 6 - 10 oder 6 - 10, 12 und 13 fortgesetzt; liegt er oberhalb oder ist er gleich 60 Gew.-% wird das Verfahren mit den Schritten 11 - 13 fortgesetzt.

### 6. Bentonitrezeptur erstellen.

Ausgehend von den in Schritt 4 ermittelten Resultaten der chemischen Analyse wird eine spezielle Bentonitrezeptur erstellt, welche einen Zuschlag von Ca-Bentonit, einem Organo-Bentonit oder einem speziellen schwermetallabsorbierenden Bentonit, wie etwa Silitonit (Fa. Südchemie, München), umfasst. Als Kriterien zur Rezepturherstellung dienen charakteristische Parameter, wie der k -Wert (Durchlässigkeitsbeiwert), die Kationenaustauschkapazität, die Schadstoffzusammensetzung und der Mineralbestand des Bodenmaterials.

### 7. Prüfung der Bentonit-Mischprobe.

Die nach dem Rezept in Schritt 6 hergestellte Mischprobe von kontaminiertem Bodenmaterial und Bentonit wird im Labor auf ihre Eignung geprüft, wobei beispielsweise bodenmechanische Versuche, Eluat-tests (nach DIN 38414-S4), oder ein Auslaugtest (nach DEV-S4) als Kriterien herangezogen werden.

### 8. Teilsanierungsfelder auskoffern (I)

Nachdem für jedes der Teilsanierungsfelder eine nach Schritt 7 geeignete Rezeptur vorliegt, werden diese in bekannter Weise ausgekoffert und die Rezeptur in einem Mixed-in-plant-Verfahren gemischt.

### 9. Überprüfung (I).

Die Ergebnisse zur Einhaltung der vorgegebenen Richtlinien werden überprüft, d.h. mit der Schadstoffparameterliste, etwa der Hollandliste, oder der Berlinerliste (Amtsblatt für Berlin, 40. Jahrgang , Nr. 65, 28. Dezember 1990, Bewertungskriterien für die Beurteilung kontaminierter Standorte in Berlin) oder ähnlichen verglichen.

Ist die Überprüfung in Schritt 9 positiv verlaufen, so wird das Verfahren mit Schritt 10 fortgesetzt.

Ist die Überprüfung in Schritt 9 negativ verlaufen, d.h. die vorgegebenen Richtlinien sind nicht oder nur teilweise eingehalten worden, so wird die Bentonitrezeptur in Schritt 6 modifiziert.

### 10. Einbau mit Gütekontrolle.

Ist die Überprüfung in Schritt 9 positiv verlaufen, d.h. die vorgegebenen Richtlinien sind eingehalten worden, so erfolgt der Einbau, bzw. die Sanierung des Teilsanierungsfeldes i nach der erstellten Rezeptur unter laufender Gütekontrolle (erdbautechnische Kontrolle), womit nach abgeschlossener Sanierung ein mit Bentonit veredeltes Bodenmaterial vorliegt, welcher die immobilisierten Schadstoffe enthält. Verläuft die Gütekontrolle negativ, so wird eine Waschung nach Schritt 12 durchgeführt.

### 11. Teilsanierungsfelder auskoffern (II)

Wurde in Schritt 4 für ein Teilsanierungsfeld ein Tonmineralgehalt grösser oder gleich 60 Gew.-% bestimmt, so wird dieses in bekannter Weise ausgekoffert.

### 12. Waschung

Die Waschung des kontaminierten Bodenmaterials, nach erfolgten Schritten 1 - 5 und 11, oder des bereits mit Bentonit veredelten, kontaminierten Bodenmaterials, nach erfolgten Schritten 1 - 10, erfolgt mit einer Alkylammonium-Lösung. Dadurch wird einerseits der Schadstoffgehalt im System Bodenmaterial-Ton reduziert, indem u.a. die Schadstoffe gegen die Alkylammoniumionen eingetauscht werden, und andererseits werden die verbleibenden Schadstoffe stärker fixiert, wodurch das Gemisch Bodenmaterial-Ton nur noch geringfügig eluiert werden kann. Bei diesem Waschprozess fällt eine leicht aufkonzentrierte Schadstofflösung an.

### 13. Überprüfung (II)

Die Effizienz der Waschung wird durch Eluatversuche (nach DEV-S4) überprüft.

Ist die Effizienz der Waschung genügend, so liegt jetzt ein veredeltes und gewaschenes Bodenmaterial vor, welcher die durch den Waschprozess erheblich verringerten und immobilisierten Schadstoffe enthält.

Ist die Effizienz der Waschung ungenügend, so wird mit einer wiederholten Waschung (Schritt 12) die Effizienz verbessert.

Für jedes Teilsanierungsfeld kann der Ablauf des Verfahrens nach der Einteilung der Sanierungsfläche (Schritt 1) unterschiedlich verlaufen. So können beispielsweise unterschiedlich viele Waschschritte (Schritt 12) notwendig werden, oder es müssen verschiedene Bentonitrezepturen (Schritt 6) erstellt werden, bis die Überprüfung (Schritt 9) ein genügendes Resultat ergibt.

Das in den Schritten 1 - 13 beschriebene Verfahren zeichnet sich durch die folgenden Vorteile aus:
a) Für jede beliebige Schadstoffzusammensetzung kann eine optimale Tonmaterialmischung zugesetzt werden.
b) Das Gemisch Bodenmaterial-Ton kann mit Proctordichte (DIN 18127) vor Ort eingebaut werden, ohne dass eine Gefahr für das umgebende Medium (Grundwasser) besteht.
c) Durch die Zugabe von Tonmaterialien und der anschliessenden Waschung mit einer organischen Lösung werden einerseits der Schadstoffgehalt im Bodenmaterial-Ton reduziert und andererseits die verbleibenden Schadstoffe stark fixiert.
d) Eine etwaige Deponierung auf einer Sondermülldeponie kann dadurch umgangen werden, da durch die minimale Eluierbarkeit nur eine Deponierung auf einer Deponie der Klasse 2 oder 3 erforderlich wird (Vorgehensweise und Rahmenbedingungen bei der Altlascensanierung in der Bundesrepublik Deutschland, MuA Lfg. 4/88, S. 282 ff. Beseitigung von Abfällen durch Ablagerung; Herausgeber Umweltbundesamt, Berlin)
Folgendes Beispiel beschreibt eine Ausführungsform zum beschriebenen Verfahren mit den Schritten 1 - 5 und 11 - 13.

Eine Sanierungsfläche von ca. 1800 m² wurde in 5 Teilsanierungsfelder von je 300 - 400 m² aufgeteilt (Schritt 1). Aus jedem dieser 5 Teilsanierungsfelder wurden in den Tiefen von 0,4 m und 0,8 m je 6 Bodenmaterialproben entnommen (Schritt 2) und daraus für jedes der Teilsanierungsfelder eine repräsentative Probe P (1) ... P(5) hergestellt (Schritt 3), welche anschliessend chemisch auf anorganische und organische Schadstoffe analysiert wurde (Schritt 4). Die Probe P (1) wies eine erhöhte Zinkkonzentration (64 ppm) auf. Für die organischen Schadstoffe wurden keine erhöhten Konzentrationen festgestellt. Die Untersuchung der Tonmaterialien ergab für die Probe P(1) folgende Zusammensetzung (in Gew.-%): Montmorillonit 54,8 %, Kaolinit 27 %, Illit 5,2 %, Carbonat 1 %, Quarz 12 %. Daraus ergab sich als Tonmineralgehalt eine Summe von 87 Gew.-% (Schritt 5), womit kein Zuschlag eines Tonmaterials erforderlich war und das Teilsanierungsfeld (1) in der üblichen Weise ausgekoffert wurde (Schritt 11). Das vorliegende Probematerial P(1) wurde mit einer 0,5 mmol Dicctadecylammoniumbromid-Lösung im Verhältnis 1:50 (Gewichtsanteile) angesetzt, gerührt und anschliessend filtriert (Schritt 12) . Als Filtrat wurde eine Schadstofflösung erhalten, in welcher noch 12 ppm Zink bestimmt wurden. Als Filterrückstand wurde ein 'Organobentonit' erhalten, der mit einem handelsüblichen Produkt verglichen wurde (Adsorption von Diethylketon), wobei keine signifikanten Unterschiede im Adsorptionsverhalten zwischen den beiden Produkten (bezogen auf Diethylketon) feststellbar waren. Zur Überprüfung der Effizienz dieses Waschprozesses (Schritt 13) wurde der 'Organobentonit' mit dem starken Komplexbildner EDTA (0,01 m EDTA-Lösung) desorbiert, was zu einer Desorptionskonzentration von 13 ppm Zink führte. Für den Eluat-Test nach DEV-S4 bedeutet dies einen viel geringeren Wert (Grenzwert kleiner 1 ppm), so dass von einem Bodenmaterial mit immobilisierten Schadstoffen gesprochen werden kann. Analog wurde mit den Proben P(2) ... P(5) verfahren, welche alle ähnliche Resultate ergaben.

Erfindungswesentlich ist, dass jedes Teilsanierungsfeld in der beschriebenen Weise optimal saniert werden kann, indem vor Ort ein Einbau erfolgt, bei welchem für das umgebende Medium (Grundwasser) keine Gefahr besteht.

Bei einem Tonmineralgehalt grösser oder gleich 60 Gew.-% werden mittels einer allenfalls mehrfach durchgeführten Waschung mit einer Alkylammoniumverbindung die Schadstoffe, organische wie auch anorganische, verringert und immobilisiert.

Bei einem Tonmineralgehalt kleiner als 60 Gew.-% wird mittels einer erstellten Bentonitrezeptur ein kontrollierter Einbau durchgeführt, wodurch die Schadstoffe immobilisert werden oder allenfalls noch zusätzlich gewaschen werden.

Das Verfahren eignet sich besonders zur Sanierung von Altlasten, wenn organische und anorganische Schadstoffe gleichzeitig vorliegen.

## Patentansprüche

1. Verfahren zur Immobilisierung von organischen und anorganischen Schadstoffen in einem kontaminierten Bodenmaterial einer Sanierungsfläche, dadurch gekennzeichnet, dass die Sanierungsfläche in Teilsanierungsfelder (i) unterteilt wird (Schritt 1), von welchen repräsentative Proben P(i) hergestellt (Schritt 3) und chemisch analysiert (Schritt 4) werden, wobei ein Tonmineralgehalt ermittelt und geprüft (Schritt 5) wird, und dass in Abhängigkeit dieses Tonmineralgehalts die Teilsanierungsfelder (i) ausgekoffert (Schritt 11), gewaschen (Schritt 12) und überprüft (Schritt 13) werden, wodurch ein Bodenmaterial mit verringerten und immobilisierten Schadstoffen und eine Schadstofflösung erhalten werden.

2. Verfahren nach Anspruch 1, gekennzeichnet dadurch, dass die Teilsanierungsfelder (i) ausgekoffert (Schritt 11) werden, wenn der ermittelte Tonmineralgehalt (Schritt 5) grösser oder gleich 60 Gew.-% ist.

3. Verfahren nach den Ansprüchen 1 - 2, gekennzeichnet dadurch, dass die Waschung (Schritt 12) mit einer Alkylammoniumlösung erfolgt.

4. Verfahren nach den Ansprüchen 1 - 3, gekennzeichnet dadurch, dass die Waschung (Schritt 12) mehrfach wiederholt wird, bis die vorgegebenen Anforderungen erfüllt sind.

5. Verfahren zur Immobilisierung von organischen und anorganischen Schadstoffen in einem kontaminierten Bodenmaterial einer Sanierungsfläche, dadurch gekennzeichnet, dass die Sanierungsfläche in Teilsanierungsfelder (i) unterteilt wird (Schritt 1), von welchen repräsentative Proben P(i) hergestellt (Schritt 3) und chemisch analysiert (Schritt 4) werden, wobei ein Tonmineralgehalt ermittelt und geprüft wird (Schritt 5), dass in Abhängigkeit dieses Tonmineralgehalts für jedes Teilsanierungsfeld (i) eine Bentonitrezeptur (Schritt 6) erstellt und eine Bentonit-Mischprobe hergestellt und geprüft wird (Schritt 7), dass die Teilsanierungsfelder (i) ausgekoffert (Schritt 8) und überprüft (Schritt 9) werden, und dass ein Einbau mittels Gütekontrolle (Schritt 10) erfolgt, wodurch ein Bodenmaterial mit immobilisierten Schadstoffen oder mittels einer Waschung (Schritt 12) ein Bodenmaterial mit verringerten und immobilisierten Schadstoffen und eine Schadstofflösung erhalten werden.

6. Verfahren nach Anspruch 5, gekennzeichnet dadurch, dass für jedes Teilsanierungsfeld (i) eine Bentonitrezeptur (Schritt 6) erstellt wird, wenn der ermittelte Tonmineralgehalt (Schritt 5) kleiner als 60 Gew.-% ist.

7. Verfahren nach den Ansprüchen 5 - 6, gekennzeichnet dadurch, dass bei ungenügendem Resultat nach der Überprüfung (Schritt 9) eine modifizierte Bentonitrezeptur erstellt (Schritt 6) wird.

8. Verfahren nach den Ansprüchen 5 - 7, gekennzeichnet dadurch, dass bei ungenügende Gütekontrolle (Schritt 10) eine Waschung (Schritt 12) mit einer Alkylammoniumlösung erfolgt.

9. Verfahren nach den Ansprüchen 5 - 8, gekennzeichnet dadurch, dass die Waschung (Schritt 12) mehrfach wiederholt wird, bis vorgegebene Anforderungen der Gütekontrolle erfüllt sind.

10. Anwendung eines Verfahrens nach einem der Ansprüche 1 - 9 zur Behandlung von Altlasten bei gleichzeitigem Vorliegen von organischen und anorganischen Schadstoffen.

## Claims

1. A process for immobilising organic and inorganic pollutants in a contaminated soil material of a remediation site wherein said remediation site is subdivided in partial remediation areas (i) (step 1) from which representative samples P (i) are prepared (step 3) and chemically analysed (step 4), while determining and checking a clay mineral content (step 5), and wherein said partial remediation areas (i) are excavated (step 11), washed (step 12) and verified (step 13) dependent on said clay mineral content, whereby a soil material with reduced and immobilised pollutants and a pollutant solution are obtained.

2. A process as in claim 1 wherein the said partial remediation areas are excavated (i) (step 11) when the premeasured clay mineral content (step 5) is 60 wt % or more.

3. A process as in claims 1 - 2 wherein the said washing (step 12) is performed using an alkyl ammonium solution.

4. A process as in claims 1 -3 wherein the said washing (step 12) is repeated several times until the specified requirements have been met.

5. A process for immobilising organic and inorganic pollutants in a contaminated soil material of a remediation site wherein said remediation site is subdivided in partial remediation areas (i) (step 1) from which representative samples P (i) are prepared (step 3) and chemically analysed (step 4), while determining and checking a clay mineral content (step 5), wherein a bentonite formulation (step 6) is developed for each partial remediation area (i) and a bentonite mixing sample is prepared and tested (step 7), wherein such partial remediation areas (i) are excavated (step 8) and tested (step 9) and wherein a quality-controlled insertion (step 10) is applied whereby a soil material with immobilised pollutants or, by means of a washing (step 12), a soil material with reduced and immobilised pollutants and a pollutant solution are obtained.

6. A process as in claim 5 wherein a bentonite formulation (step 6) is prepared for each partial remediation area (i) when the pre-measured clay mineral content (step 5) is less than 60 wt %.

7. A process as in claims 5 - 6 wherein a modified bentonite formulation is developed (step 6) in the event that the result after retesting (step 9) is inadequate.

8. A process as in claims 5 - 7 wherein a washing with an alkyl ammonium solution (step 12) is carried out in case of inadequate quality control (step 10).

9. A process as in claims 5 - 8 wherein the said washing (step 12) is repeated several times until the specified requirements of quality control are met.

10. An application of a process as in claims 1 - 9 for the treatment of abandoned contaminated sites containing organic and anorganic pollutants at the same time.

## Revendications

1. Procédé pour immobiliser des matières nocives organiques et inorganiques dans un matériau de sol contaminé d'une surface d'assainissement caractérisée par le fait que la surface d'assainissement est subdivisée en zones partielles d'assainissement (i) (étape 1) desquelles des échantillons représentatifs P(i) sont fabriqués (3) et analysés chimiquement (étape 4). Ce faisant, une teneur en minéral des argiles réfractaires est déterminé et contrôlé (étape 5). En fonction de cette teneur, les zones partielles d'assainissement (i) sont décoffrées (étape 11), lavées (étape 12) et vérifiées (étape 13) moyennant quoi, on obtient un matériau de sol comportant une quantité réduite de matières nocives immobilisées et une solution de matières nocives.

2. Procédé selon la revendication 1 caractérisé par le fait que les zones partielles d'assainissement (i) sont décoffrées (étape 11), lorsque la teneur en minéral des argiles réfractaires déterminé (étape 5) est supérieure ou égale à 60 % en poids.

3. Procédé selon les revendications 1 - 2 caractérisé par le fait que le lavage (étape 12) a lieu avec une solution d'alkylammonium.

4. Procédé selon les revendications 1 - 3 caractérisé par le fait que le lavage (étape 12) est répété plusieurs fois jusqu'à ce que les exigences posées soient remplies.

5. Procédé pour immobiliser des matières nocives organiques et inorganiques dans un matériau de sol contaminé d'une surface d'assainissement caractérisée par le fait que la surface d'assainissement est subdivisée en zones partielles d'assainissement (i) (étape 1) desquelles des échantillons représentatifs P(i) sont fabriqués (3) et analysés chimiquement (étape 4). Ce faisant, une teneur en minéral des argiles réfractaires est déterminé et contrôlé (étape 5) . En fonction de cette teneur pour chaque zone partielle d'assainissement (i) une formulation de bentonite (étape 6) est établie et un spécimen composite de bentonite est fabriqué et contrôlé (étape 7), les zones partielles d'assainissement (i) sont décoffrées (étape 8) et vérifiées, puis une incorporation a lieu au moyen d'un contrôle de qualité (étape 10) moyennant quoi, on obtient un matériau de sol comportant des matières nocives immobilisées ou au moyen d'un lavage (étape 12), un matériau de sol comportant une quantité réduite de matières nocives immobilisées et une solution de matières nocives.

6. Procédé selon la revendication 5 caractérisé par le fait que pour chaque zone partielle d'assainissement (i) une formulation de bentonite (étape 6) est établie lorsque la teneur en minéral des argiles réfractaires déterminé (étape 5) est inférieur à 60 % en poids.

7. Procédé selon les revendications 5 - 6 caractérisé par le fait que dans le cas d'un résultat insuffisant après vérification (étape 9), une formulation de bentonite modifiée est établie (étape 6).

8. Procédé selon les revendications 5 - 7 caractérisé par le fait que dans le cas d'un contrôle de qualité insuffisant (étape 10), un lavage (étape 12) a lieu avec une solution d'alkylammonium.

9. Procédé selon les revendications 5 - 8 caractérisé par le fait que le lavage (étape 12) est répété plusieurs fois jusqu'à ce que les exigences posées du contrôle de qualité soient remplies.

10. Application d'un procédé selon les revendications 1 - 9 pour le traitement de vieilles charges en présence de matières nocives organiques et inorganiques.
